# EUROPEAN PATENT SPECIFICATION

(11) **EP 3 079 584 B1**
(45) Date of publication and mention of the grant of the patent: **11.10.2017**
(21) Application number: 14835674.4
(22) Date of filing: 11.12.2014
(51) Int. Cl.: A61B 5/113, A61B 6/03, A61B 6/00, A61B 6/12, A61B 5/00, A61B 17/00, A61B 5/055

(54) **METHOD AND SYSTEM FOR RESPIRATORY MONITORING DURING IMAGE GUIDED INTERVENTIONAL PROCEDURES**
VERFAHREN UND SYSTEM ZUR ATEMÜBERWACHUNG BEI BILDGEFÜHRTEN INTERVENTIONELLEN VERFAHREN
PROCÉDÉ ET SYSTÈME DE SURVEILLANCE RESPIRATOIRE DURANT DES INTERVENTIONS GUIDÉES PAR IMAGE

(30) Priority: 12.12.2013 US 201361915155 P
(43) Date of publication of application: 19.10.2016
(73) Proprietor: Koninklijke Philips N.V., 5656 AE Eindhoven (NL)
(72) Inventor: GOTMAN, Shlomo, NL-5656 AE Eindhoven (NL)
(74) Representative: Versteeg, Dennis John
(86) International application number: PCT/IB2014/066800
(87) International publication number: WO 2015/087273

(56) References cited:
- WO-A1-2013/013142
- JP-A- 2000 070 259
- US-A1- 2003 114 749
- US-A1- 2003 188 757
- US-A1- 2010 063 514

## Description

The present application relates generally to a method and system for respiration-based guidance during interventional procedures. Interventional procedures can include interactive image-guided surgery and interactive surgical procedures, such as biopsies. It finds particular application with minimally invasive surgical procedures performed in conjunction with x-ray computed tomography (CT) imaging systems. These procedures involve the use of surgical tools for biopsy or brachytherapy needles or the like for tissue sampling or planning or placement of objects or instruments within the body of a subject, such as a patient. It is to be appreciated, however, that the invention is also applicable to a wide range of other imaging equipment and techniques, for example ultrasonic and magnetic resonance imaging devices, PET, SPECT, etc., and to a broad range of minimally invasive surgical procedures including many forms of surgery for placing objects or instruments at precise locations within a patient, such as interventional radiology procedures and others. A typical goal of interventional procedures, along with most procedures involving radiation, is to control the radiation delivery to minimize radiation exposure to the patient and the technician, such as a radiologist.

A CT scanner is commonly used for image guidance during interventional procedures. While it is possible to continuously watch the needle advancement using CT fluoroscopy, this method is seldom used due to much higher radiation dose for the patient, hand exposure to the primary beam for the radiologist, and inconvenience to manipulate the tool, such as, for example, a needle, inside a CT gantry bore. Therefore, a common practice is to use incremental tool advancement with periodic verification of the tool position by a single CT shot or scan.

One of the major challenges during such procedures is respiratory motion. Due to such motion, the position of the internal organs in the target area during tool manipulation can differ significantly from the position of the internal organs during the prior CT scan.

Respiratory monitoring has been used in the context of CT-guided procedures to select the optimal time for a CT scan, primarily to avoid motion artifacts (e.g., by using respiratory gating) or for post-procedure analysis of the recorded respiration wave (e.g., for radiation therapy planning).

One common practice used to address respiratory motion during an interventional procedure is to have the patient hold his breath at the same level during both the CT scan and afterwards, during the intervention tool insertion. However, this approach has distinct disadvantages: reproducibility of diaphragm position between breath-holds is not good; and after a breath-hold, the following deep respiration may cause tool laceration to the patient, such as, for example, to the pleura. In addition, some patients (especially with pulmonary problems) have difficulty holding their breath. A method and a system according to the preamble of independent claims 1 and 12 are known from US2003/0114749. The proposed method and device allows a user, such as, for example, a technician, radiologist, or surgeon, to manipulate the interventional tool at times when the positions of the internal organs are close to their positions during a prior CT scan. The method is based on continuous respiratory monitoring of the patient, both during the scan and during tool advancement during the interventional procedure, with, for example, audio or visual notification of when to start and/or stop the tool manipulation, for example, needle advancement. The invention is defined by the independent claims 1 and 12. In one embodiment, a respiratory monitoring method for indicating intervention tool advancement timing during an intervention procedure includes monitoring respiration of a subject using a respiration monitor, wherein the respiration monitor produces a respiratory signal indicative of a plurality of respiratory states of the subject, scanning the subject using an imaging device and generating a first imaging data set, wherein the first imaging data set is associated with a first respiratory state of the subject, and indicating when to advance an intervention tool based on the respiratory signal using an advancement indicator, such that advancement of the intervention tool occurs during the first respiratory state of the subject, wherein the respiration of the subject is continuous.

Numerous advantages and benefits will become apparent to those of ordinary skill in the art upon reading the following detailed description of several embodiments. The invention may take form in various components and arrangements of components, and in various process operations and arrangements of process operations. The drawings are only for the purpose of illustrating many embodiments and are not to be construed as limiting the invention.

The descriptions of the invention do not limit the words used in the claims in any way or the scope of the claims or invention. The words used in the claims have all of their full ordinary meanings.

In the accompanying drawings, which are incorporated in and constitute a part of the specification, embodiments of the invention are illustrated, which, together with a general description of the invention given above, and the detailed description given below, serve to exemplify embodiments of this invention.
FIGURE 1 illustrates an exemplary CT imaging system with an exemplary intervention tool advancement indicator system;
FIGURE 2 illustrates an exemplary integrated apparatus with exemplary respiratory monitoring, imaging, and interventional procedure systems;
FIGURE 3 is a schematic representation of an exemplary system for respiratory monitoring and indicating intervention tool advancement timing during an intervention procedure;
FIGURE 4 shows the configuration of an exemplary scanner and intervention tool advancement indicator during the scanning stage of an exemplary intervention procedure;
FIGURE 5 shows the configuration of an exemplary scanner and intervention tool advancement indicator during the intervention tool advancement stage of an exemplary intervention procedure;
FIGURE 6 shows an exemplary waveform representing a patient's respiration state and triggering points for an intervention tool advancement indicator;
FIGURE 7 shows an exemplary bar graph representing a patient's respiration state and a triggering point for an intervention tool advancement indicator;
FIGURE 8 shows another exemplary waveform representing a patient's respiration state and triggering points for an intervention tool advancement indicator;
FIGURE 9 shows another exemplary waveform representing a patient's respiration state and triggering points for an intervention tool advancement indicator;
FIGURE 10 is a flowchart of an exemplary method of indicating when to advance an intervention tool;
FIGURE 11 is a flowchart of an exemplary method of advancing an intervention tool;
FIGURE 12 is a flowchart of another exemplary method of indicating when to advance an intervention tool;
FIGURE 13 is a flowchart of an exemplary method of selecting an image data set for use during an intervention procedure; and
FIGURE 14 is a flowchart of an exemplary method of developing a plan for indicating when to advance an intervention tool.

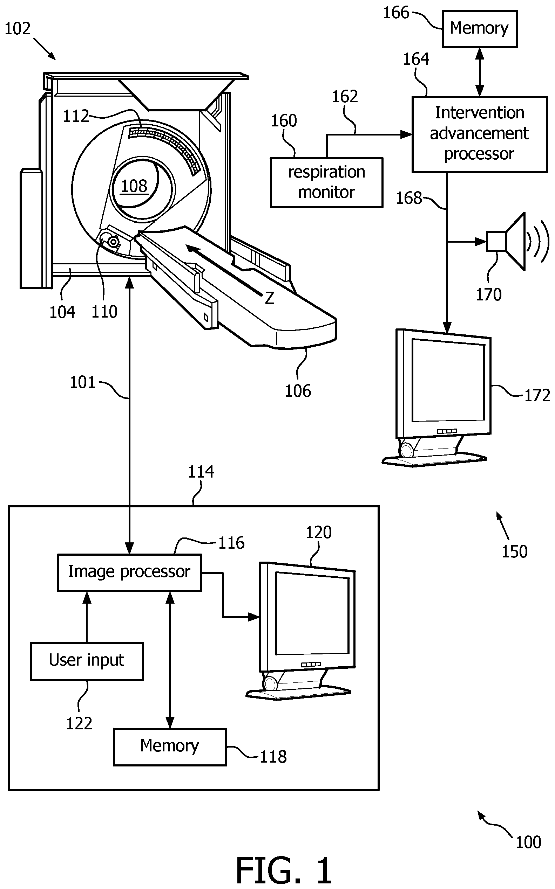

In one embodiment, an exemplary CT imaging system 100 and an exemplary intervention tool advancement indicator system 150 are shown in FIGURE 1. A CT imaging acquisition system 102 includes a gantry 104 and a table or other support 106 which may move along the z-axis. A patient or other subject to be imaged (not shown in FIGURE 1) lies down on the table 106 and is moved to be disposed within an aperture or bore 108 in the gantry 104. Once the patient is in position, an x-ray source 110 and an x-ray detector 112 rotate together around the bore 108 to record CT imaging data. Other imaging system modalities may also be used in conjunction with the claimed invention, including, for example, cone beam CT, other x-ray based imaging, ultrasound imaging, magnetic resonance imaging (MRI), positron emission tomography (PET) imaging, and the like.

The CT imaging acquisition system 102 can then pass the CT imaging data on to a CT imaging processing and display system 114 through a communication link 101. Although the systems 102 and 114 are shown and described here as being separate systems for purposes of illustration, they may in other embodiments be part of a single system. The CT imaging data passes to an image processor 116 which can store the data in a memory 118. The image processor 116 electronically processes the data to perform an image reconstruction. The image processor 116 can show the resulting images on an associated display 120. A user input 122 such as a keyboard and/or mouse device may be provided for a user to control the processor 116.

An exemplary intervention tool advancement indicator system 150 is also shown in FIGURE 1. The indicator system 150 includes a respiration sensor or monitor 160 that monitors an aspect of the subject's respiration. The respiration monitor 160 can provide a respiration signal 162 to an intervention advancement processor 164, which can store data and other information in a memory 166. The intervention advancement processor 164 may include logic for determining when to produce an advancement indicator signal 168. The advancement indicator signal 168 may be used to drive one or more indicating devices, such as, for example, an audible indicator, such as speaker 170, and/or a visual indicator, such as display 172.

Many of the aforementioned functions can be performed as software logic. "Logic," as used herein, includes but is not limited to hardware, firmware, software and/or combinations of each to perform a function(s) or an action(s), and/or to cause a function or action from another component. For example, based on a desired application or needs, logic may include a software controlled microprocessor, discrete logic such as an application specific integrated circuit (ASIC), or other programmed logic device. Logic may also be fully embodied as software.

"Software," as used herein, includes but is not limited to one or more computer readable and/or executable instructions that cause a computer, processor, or other electronic device to perform functions, actions, and/or behave in a desired manner. The instructions may be embodied in various forms such as routines, algorithms, modules or programs including separate applications or code from dynamically linked libraries. Software may also be implemented in various forms such as a stand-alone program, a function call, a servlet, an applet, instructions stored in a memory such as memories 118 and 166, part of an operating system or other type of executable instructions. It will be appreciated by one of ordinary skill in the art that the form of software is dependent on, for example, requirements of a desired application, the environment it runs on, and/or the desires of a designer/programmer or the like.

The systems and methods described herein can be implemented on a variety of platforms including, for example, networked control systems and stand-alone control systems. Additionally, the logic shown and described herein preferably resides in or on a computer readable medium such as the memory 118 and/or 166. Examples of different computer readable media include Flash Memory, Read-Only Memory (ROM), Random-Access Memory (RAM), programmable read-only memory (PROM), electrically programmable read-only memory (EPROM), electrically erasable programmable read-only memory (EEPROM), magnetic disk or tape, optically readable mediums including CD-ROM and DVD-ROM, and others. Still further, the processes and logic described herein can be merged into one large process flow or divided into many sub-process flows. The order in which the process flows herein have been described is not critical and can be rearranged while still accomplishing the same results. Indeed, the process flows described herein may be rearranged, consolidated, and/or re-organized in their implementation as warranted or desired.

The exemplary intervention tool advancement indicator system 150 may be a stand-alone system or in other embodiments may be fully or partially integrated with the exemplary CT imaging system 100 to form a combined system.

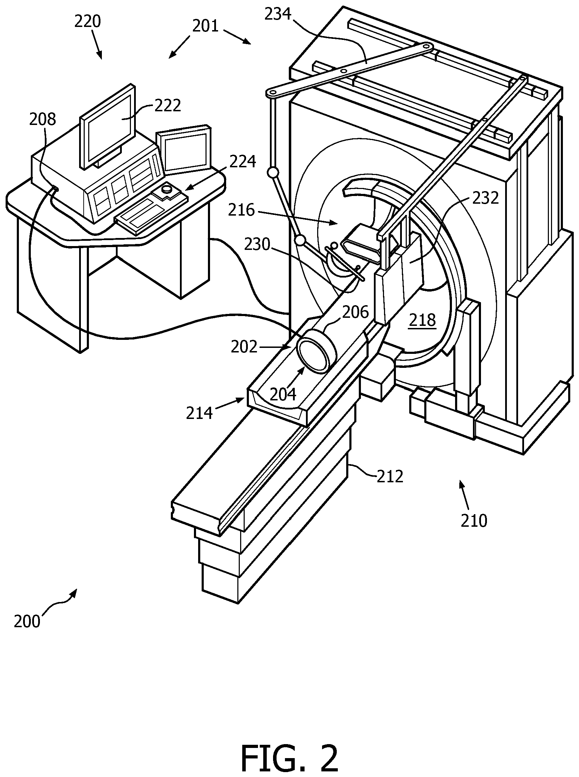

Referring now to FIGURE 2, another embodiment is shown with an exemplary integrated apparatus 200, which includes an exemplary respiratory monitor system 202 and an exemplary imaging system 201. The integrated apparatus 200 is particularly well suited for planning and executing minimally invasive interventional procedures, such as, for example, biopsies, in-vivo placement of instruments and/or objects within a patient, etc., as described above.

The respiratory monitor system 202 includes a respiratory sensor 204. In one embodiment, the respiratory sensor 204 may be a belt 206 adapted for attachment around the abdomen or chest of a patient. In one embodiment, the respiratory sensor 204 includes an air bellows sensor and pressure transducer (not shown) for generating a signal corresponding to the displacement of a patient's abdomen during respiration. The respiratory sensor 204 is attached to the imaging system 201 at a suitable electronic connection point 208.

With continued reference to FIGURE 2, the imaging system 201 includes a volumetric diagnostic CT imaging apparatus/scanner 210 as shown. The CT imaging apparatus 210 is disposed in axial alignment with a patient table 212 and support 214 such that a patient or subject on the support 214 surface can be moved into and through a bore 216 of the CT scanner 210. The CT scanner 210 includes an x-ray tube mounted for rotation about a preselected plane. The x-ray tube can project a fan shaped beam of radiation through a ring 218 of radiation translucent material, through the patient support 214, through a region of interest or target area of the patient, and to a ring or arc of radiation detectors disposed opposite the x-ray tube. As the x-ray tube rotates within the plane, a series of data lines are generated, which data lines are reconstructed into at least a slice image using well known techniques by a reconstruction processor included in a control console 220 operatively connected with the CT scanner 210.

As is well known in the art, in some embodiments, the patient support 214 can move longitudinally along the z-axis as the x-ray tube is rotating around the subject such that a selected volume of the patient is scanned along a spiral path or a series of slices. The position of the x-ray tube is monitored by a rotational position encoder and the longitudinal position of the patient support is monitored by similar position encoders disposed within the table 212. In other embodiments, volumetric data may be obtained without longitudinal movement. The reconstruction processor can reconstruct a volumetric image representation from the generated data lines. The control console 220 includes one or more human readable display devices, which can be in the form of an operator monitor or display 222 and at least one operator input device 224, such as, for example, a keyboard, track ball, mouse, or the like.

With continued reference to FIGURE 2, an exemplary intervention tool 230 is shown above the patient support 214. An exemplary intervention tool advancement indicator device 232 is shown opposite the intervention tool 230. The intervention tool advancement indicator device 232 may include, for example, an audible indicator, such as speaker 170, and/or a visual indicator, such as display 172, as shown in FIGURE 1. The intervention tool 230 and intervention tool advancement indicator device 232 are shown supported from overhead on a track or by other means 234 atop the CT scanner 210. In this embodiment, the intervention tool 230 and intervention tool advancement indicator device 232 are shown in exemplary positions that may be convenient for a user performing an exemplary interventional procedure on the subject. However, the positioning of any of the devices shown in this embodiment may be arranged or supported differently in other embodiments. The intervention tool advancement indicator device 232 can be oriented or moved into selected positions on the support system 234 for easy viewing and/or hearing by a user.

As shown in FIGURE 2, the exemplary control console 220 may include both an image processor and an intervention advancement processor (not shown), such as, for example, the image processor 116 and the intervention advancement processor 164 of FIGURE 1. In various embodiments, these processors may be physically and/or electronically integrated within the control console 220 and/or each other. In another embodiment, the intervention advancement processor 164 may be included in a device with the intervention tool advancement indicator device 232.

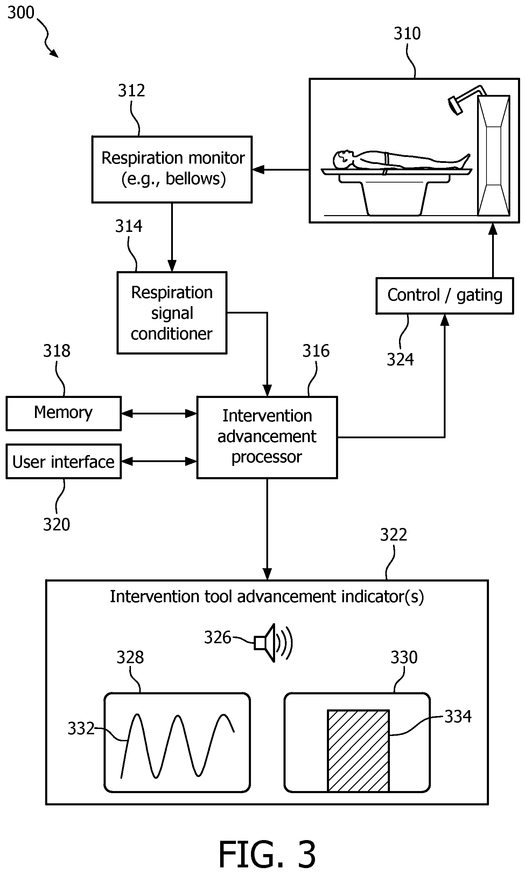

FIGURE 3 is a schematic representation of an exemplary system 300 for respiratory monitoring and indicating intervention tool advancement timing during an intervention procedure. System 300 includes an exemplary scanning system 310, such as, for example, a CT scanner. The system 300 further includes an exemplary respiration monitor 312, such as, for example, a bellows belt, positioned to monitor the respiration of a patient.

The respiratory signal from the respiration monitor 312 may be conditioned by a respiration signal conditioner 314. In one embodiment, the respiration signal conditioner 314 includes a respiratory sensor circuit (not shown) that includes a pressure transducer responsive to a condition of the air bellows as well as an amplification circuit for amplifying the electrical signal from the pressure sensor to a level suitable for input to an analog to digital converter circuit. The conversion of analog signals from the belt representative of a position of the patient's abdomen to a digital signal for use by a processing unit is well known in the art and can be accomplished using any suitable equivalent means.

As illustrated, an intervention advancement processor 316 receives the respiratory signal from the respiration monitor 312 or conditioner 314 and is operatively connected with several components of the system 300, including a memory 318, a user interface 320, one or more intervention tool advancement indicators 322, and a scanning system controller/gating device 324. The memory 318 may be used to store various software, logic, and/or parameters utilized by the intervention advancement processor 316, including, for example, values and parameters associated with respiration states of the subject, associated intervention tool triggering/threshold values and/or levels, algorithms for determining triggering/threshold points and/or levels, user selected values, image data, etc., as discussed in more detail below. The user interface 320 can include user input and display devices and may be integrated as part of control console 220 of FIGURE 2.

The exemplary intervention tool advancement indicators 322 may include one or more audible indicators, such as speaker 326, and/or one or more visual indicators, such as displays 328, 330. The speaker 326 can produce a continuous sound, instances of the same sound, or instances of different sounds to indicate when to start and stop the intervention tool advancement. For example, a continuous beep or "on" and "off" beeps may be used for indicating when to advance the intervention tool.

The displays 328, 330 can include visual indicia of the patient's breathing and/or indicate when to advance the intervention tool, as discussed in more detail below. In one embodiment, a display 328, 330 may simply display a visual cue, such as, for example, a word or color (e.g., red and/or yellow followed by green) to indicate when to advance the intervention tool. In other embodiments, as shown in FIGURE 3, the displays 328, 330 can depict a representation of the patient's breathing. Exemplary display 328 shows a waveform 332 having an amplitude representing the respiration of the patient. Exemplary display 330 shows a bar graph 334 having a height representative of an inhalation/exhalation level of the patient on a scale of percentage of vital capacity (% VC). In one embodiment, in order to adjust for variations of placement of the respiration monitor 312 on the patient, gains and affects are applied based on vital capacity for display purposes. It is to be appreciated that although a waveform and bar graph are illustrated, other forms of patient breathing images can be used as well such as, for example, a graduated cylinder, a progress bar, an animated diaphragm, and the like.

In addition to driving intervention tool advancement indicators 322, the intervention advancement processor 316 can be used to provide a signal to be used to control the scanning system 310. For example, control/gating device 324 may be used to control or provide a signal to the the scanning system 310 to scan the patient at a particular respiration state, for example, at the same respiration state during which the user was advancing the intervention tool. This may be helpful during confirmation scans. In one embodiment, the control/gating device 324 may be integrated as part of control console 220 of FIGURE 2.

Generally, the system 300 tracks the respiration of the patient using the respiration monitor/signal and indicates to the user when to advance the intervention tool. In particular, the intent is to advance the intervention tool when the patient's respiration state is at the same state as when the patient was scanned to produce the image data set. As discussed above, the patient's internal organs and chest cavity may move during respiration, but this movement can be correlated to the patient's respiration states. It is beneficial and preferable to advance the intervention tool when the user knows where the patient's internal organs and chest cavity are positioned. Therefore, knowing the respiration state of the patient during the scan, which is the source of the image data that the user uses to guide the intervention tool during intervention tool advancement, allows the system 300 to indicate to the user when the patient's internal organs and chest cavity will be in the same position again, i.e., when the patient is in the same respiration state.

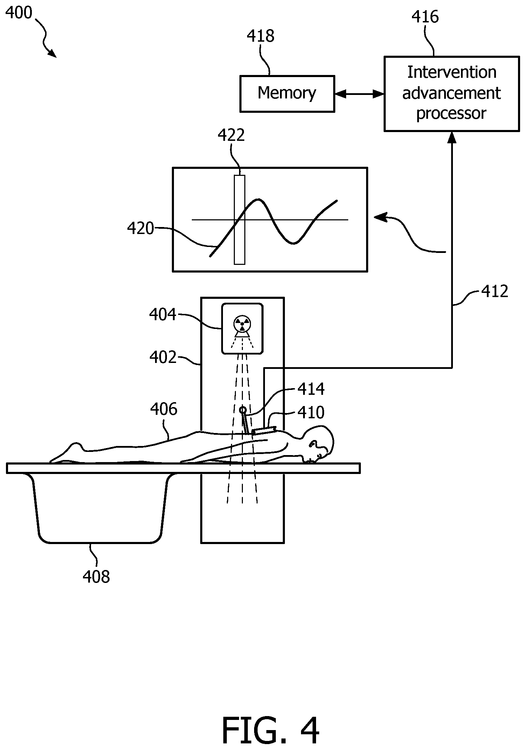

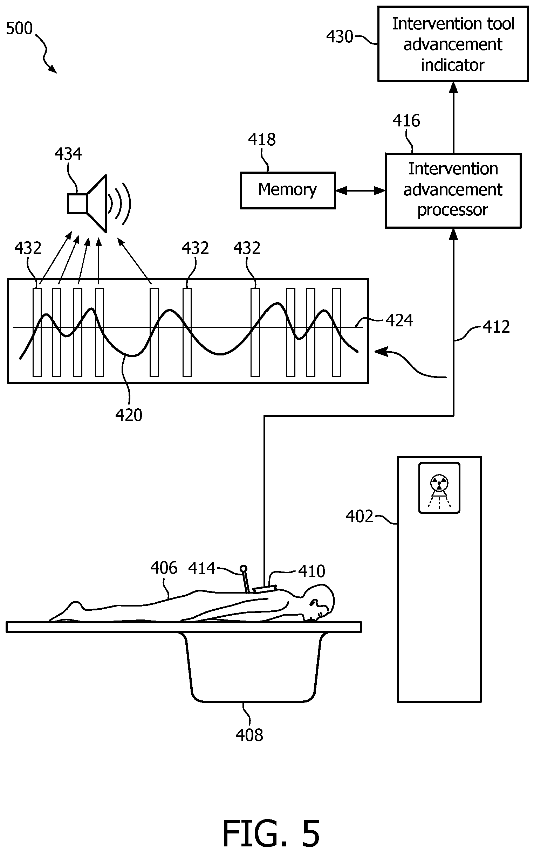

FIGURES 4 and 5 show the configurations of an exemplary scanner and intervention tool advancement indicator during the different stages of an exemplary intervention procedure. Referring first to FIGURE 4, an exemplary scanning stage 400 is shown. During the scanning stage 400, an exemplary scanner 402 with radiation device 404 is shown scanning a patient 406 on support table 408. A respiration monitor 410 monitors a characteristic of the patient's normal breathing pattern during the scan, producing a respiration signal 412 indicative of the patient's breathing states. It is important to note that the patient 406 can breathe normally and continuously during the procedure. The patient 406 does not have to hold his breath.

The scanner 402 produces an image data set, such as, for example, a volumetric data set, suitable to assist and guide the user during the subsequent intervention procedure. Exemplary image processing devices are not shown in FIGURE 4, but may be as described above in FIGURES 1 and 2. An exemplary intervention tool 414 is also shown as being scanned with the target area of the patient 406, although this need not be the case in all embodiments. As shown in this embodiment, it may be helpful to have the intervention tool 414 scanned with the patient 406 to establish a reference starting position of the intervention tool on the scan and in the image data set. During stage 400, one or more scans may be taken of the patient 406. For each scan, an intervention tool advancement processor 416 reads the respiration signal 412 and associates the signal reading with the respective image data set. The respiration signal 412 is also associated with a particular respiration state of the patient 406. All of this data may be saved in memory 418. To graphically represent what is happening during the scan stage 400, waveform 420 is shown representing the respiration signal 412 during the patient's normal breathing. When the scanner 402 scans the patient, the respiration state 422 of the patient 406, defined by the respiration signal 412, is captured and associated with the image data set from the scan.

As shown in FIGURE 5, an exemplary intervention tool advancement stage 500 is shown, which follows a scanning stage 400. During the tool advancement stage 500, the patient 406 is removed from the scanner 402, allowing for intervention tool advancement without additional exposure to radiation from the scanner 402. The respiration monitor 410 continues to monitor the characteristic of the patient's normal breathing pattern and produce the respiration signal 412 indicative of the patient's breathing states. The patient 406 can still breathe normally and continuously during the intervention tool advancement stage 500; the patient 506 does not need to hold his breath. The intervention advancement processor 416 drives an intervention tool advancement indicator 430 that indicates when the patient's respiration state is at the same state 422 as it was during the scan that created the image data set being used by the user to guide advancement of the intervention tool 414.

In this manner, the user is alerted to when the patient's internal organs and chest cavity are in the proper position to advance the intervention tool 414, by tracking the patient's respiration. In particular, the intervention advancement processor 416 can monitor the patient's respiration state for certain thresholds/triggering points to drive the intervention tool advancement indicator 430, which indicates to the user a suitable time to advance the intervention tool 414. To graphically represent what is happening during the tool advancement stage 500, waveform 420 is shown representing the respiration signal 412 during the patient's normal breathing. Line 424 represents the respiration signal level corresponding to the level associated with the image data set and the associated respiration state 422 of the patient 406 from scan stage 400. Each time the respiration signal 412 is at (i.e., crosses) line 424, the patient's respiration state (and the position of the patient's internal organs and chest cavity) corresponds to the respiration state (and position) associated with the image data set. Based on these crossing points 432, the intervention tool advancement indicator 430 provides an indication to the user (e.g., via speaker 434) that it is a suitable time to advance the intervention tool 414, because the user knows that the patient's target area should be in a position matching the image data set.

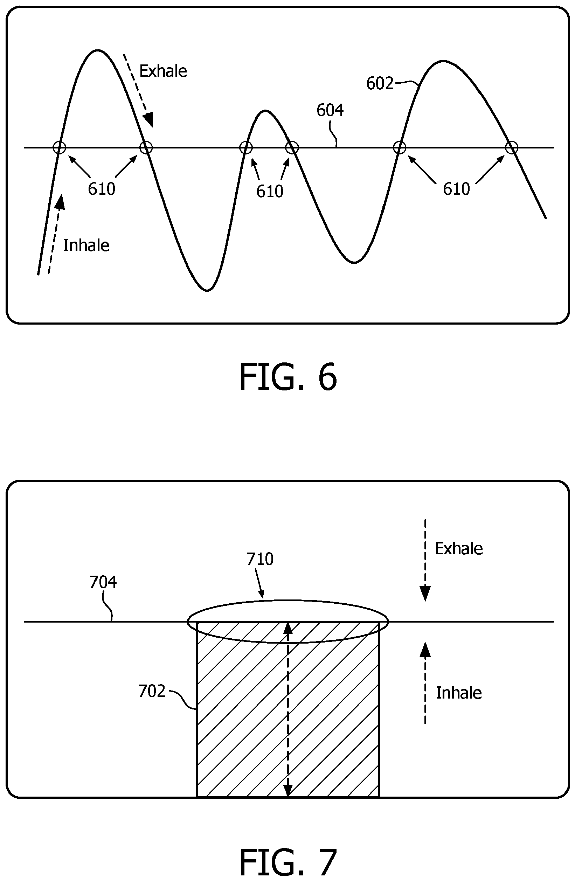

FIGURES 6-9 show exemplary graphical representations of a patient's respiration states used for triggering an intervention tool advancement indicator. The representations shown in FIGURES 6-9 may be representative of the tracking and triggering algorithms utilized by an intervention advancement processor, which in some embodiments may also be displayed on a display associated with an intervention tool advancement indicator (e.g., display 172 in FIGURE 1, displays 222, 232 in FIGURE 2, and displays 332, 334 in FIGURE 3).

FIGURE 6 shows an exemplary waveform 602 representing a patient's respiration state during normal breathing, but with variations in time and lung capacity fill levels from one breath to another. Waveform 602 rises and falls over time while the patient inhales and exhales, respectively. Target line 604 represents the respiration state corresponding to an associated image data set for use during the intervention procedure and from an earlier scan. Each time the patient's real-time respiration state 602 is at (i.e., crosses) line 604, the patient's respiration state (and the position of the patient's internal organs and chest cavity) corresponds to the respiration state (and position) associated with the image data set. In this embodiment, the intervention advancement processor uses these crossing points as triggering points 610 to drive an intervention tool advancement indicator, which indicates to the user a suitable time to advance an intervention tool. In one embodiment, the intervention tool advancement indicator provides the same indication (e.g., "beep" sound) for triggering points 610 that occur during an inhale and during an exhale. In another embodiment, the intervention tool advancement indicator provides different indication (e.g., different "beep" sounds) for triggering points 610 that occur during an inhale and during an exhale.

The intervention advancement processor may use a variety of techniques for determining the length of time to drive the indicator. In one embodiment, the time (or window of advancement) may be user-selectable based on the specifics of the intervention procedure, such as, for example, the sensitivity and/or type of procedure, the proximity of the intervention tool to other organs and their associated sensitivity, the type of intervention tool, user preference, etc. In different embodiments, determining the length of time to drive the indicator may be based on a model formed on organ movement, based on the location of the tool, and/or based on an intervention procedure plan that can change the length of time to drive the indicator during different phases of the procedure and/or based on procedure progress, etc. In one embodiment, the processor may drive the indicator for a predefined time after the trigger point 610. In another embodiment, the processor may drive the indicator for a variable time after the trigger point 610, which is based on the slope of the waveform 602 as it approaches or crosses the target line 604. FIGURE 7 shows an exemplary bar graph 702 having a height representative of an inhalation/exhalation level of the patient on a scale of percentage of vital capacity (% VC) during normal breathing (i.e., patient not holding breath). In other embodiments, other forms of patient breathing images can be used as well, such as, for example, a graduated cylinder, a progress bar, an animated diaphragm, and the like.

Bar graph 702 rises and falls over time while the patient inhales and exhales, respectively. Target line 704 represents the respiration state corresponding to an associated image data set for use during the intervention procedure and from an earlier scan. Each time the patient's real-time respiration state 702 is at (i.e., crosses) line 704, the patient's respiration state (and the position of the patient's internal organs and chest cavity) corresponds to the respiration state (and position) associated with the image data set. In this embodiment, the intervention advancement processor uses these crossing points as triggering points 710 to drive an intervention tool advancement indicator, which indicates to the user a suitable time to advance an intervention tool.

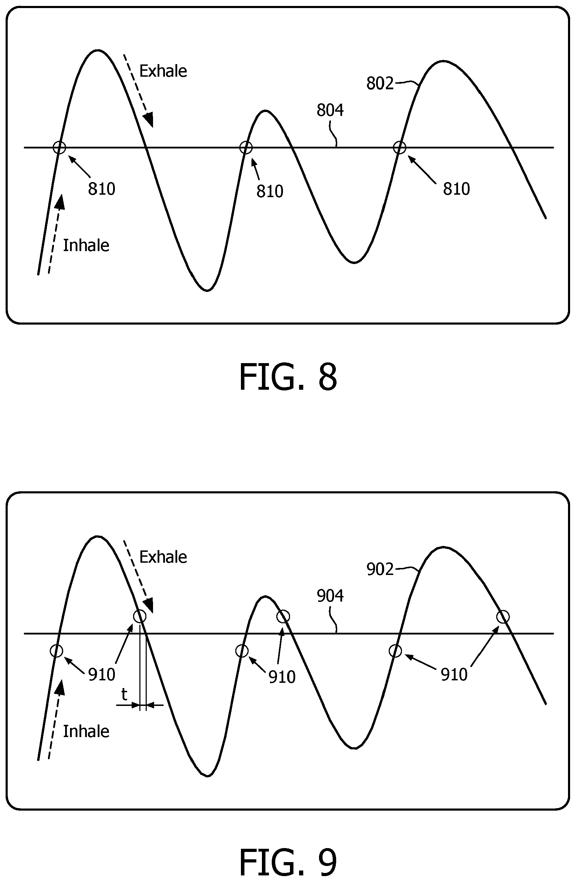

FIGURE 8 shows another exemplary waveform 802 representing a patient's respiration state. Target line 804 represents the target respiration state corresponding to an associated image data set for use during the intervention procedure and from an earlier scan. In this embodiment, triggering is limited to each time the patient's real-time respiration state 802 is at (i.e., crosses) line 804 during an inhalation period of the patient's respiration. The intervention advancement processor uses only these crossing points during inhalation as triggering points 810 to drive an intervention tool advancement indicator.

In other embodiments, triggering points may be limited to exhalation periods or any other feature of the patient's respiration. In yet other embodiments, the triggering points can be further based on other factors, such as, for example, time, physiological characteristics of the patient, non-physiological factors, etc. For example, besides satisfying the respiration state requirement, a triggering point may be limited to only occur a minimum time after the previous trigger point, within certain waveform slope ranges, within certain patient heart rate ranges, within certain patient blood pressure ranges, etc.

FIGURE 9 shows another exemplary waveform 902 representing a patient's respiration state. Target line 904 represents the target respiration state corresponding to an associated image data set for use during the intervention procedure and from an earlier scan. In this embodiment, the intervention advancement indicator is triggered in anticipation of the expected occurrence of the target respiration state 904. The intervention advancement processor can use an algorithm to trigger the intervention advancement indicator a certain time t before the target respiration state 904 is reached. This time t may be used to provide the user with a time t to prepare for advancing the intervention tool. As shown in FIGURE 9, the indicator is triggered at trigger points 910, which occur at a time t before the respiration state 904 is expected to be reached. The algorithm utilizes the slope of the waveform 902 as it approaches the target line 904 to determine when to trigger the advancement indicator at points 910 to provide the requisite time t before the respiration state 904 is reached.

The intervention advancement processor may use a variety of techniques for determining the time t. In various embodiments, the time t may be user-selectable based on user preference and/or the specifics of the intervention procedure, such as, for example, the amount of advancement during each trigger point, the type of intervention tool, etc.

In other embodiments, separately or in combination with the time t, the duration of the indicator (i.e., indicator time) may be adjusted. For example, the indicator may turn off at a predefined amount of time (e.g., user selectable) after it is turned on (the trigger point). In other embodiments, the indicator may turn off at a variable time after the trigger point, which may be based on the slope or other characteristics of the waveform before, during, and/or after it crosses the target line. For example, in one embodiment, the processor may monitor the respiration waveform and turn on the intervention indicator at a time t before the target respiration state is reached and turn off the indicator at a time t after the target respiration state is reached. As can be appreciated, many other techniques and combinations of trigger on and trigger off algorithms can be utilized.

Various parameters and algorithms associated with the intervention tool advancement indicator, such as, start time, stop time, length of time to drive the indicator, responses to intervention, patient, and/or outside factors, etc., may be determined as part of a planning phase to develop an intervention procedure plan, which typically precedes the intervention procedure. In some embodiments, the intervention procedure plan may be modified during the intervention procedure, based on various factors, including, for example, a confirmation scan.

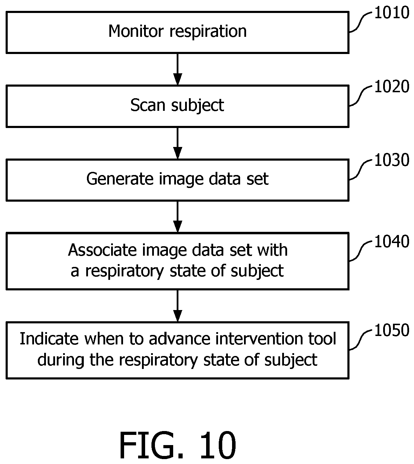

FIGURES 10-14 describe exemplary methods associated with utilization of intervention tool advancement indicator systems, including, for example, those mentioned above. Further embodiments of similar methods may include other additional steps, or omit one or more of the steps in the illustrated methods. Also, the order in which the process flows herein have been described may be rearranged while still accomplishing the same results. Thus the process flows described herein may be added to, rearranged, consolidated, and/or re-organized in their implementation as warranted or desired.

FIGURE 10 is a flowchart of an exemplary method of indicating when to advance an intervention tool while the subject (e.g., patient) can breathe normally and continuously, without holding his breath. At step 1010, a patient's respiration is monitored using a respiration monitor. Next, while monitoring the patient's respiration, the patient is scanned using a scanner at step 1020 and an image data set is generated from the scan at step 1030. At step 1040, the image data set is associated with the respiratory state of the patient at the time of the scan. At step 1050, while continuing to monitor the patient's respiration, an intervention tool advancement indicator can indicate when to advance an intervention tool during a subsequent respiratory state of the patient that matches the respiratory state of the image data set. In this manner, the advancement indicator can indicate when to advance the intervention tool when the patient's target area is in the same position as when the scan was taken and the patient does not need to hold his breath.

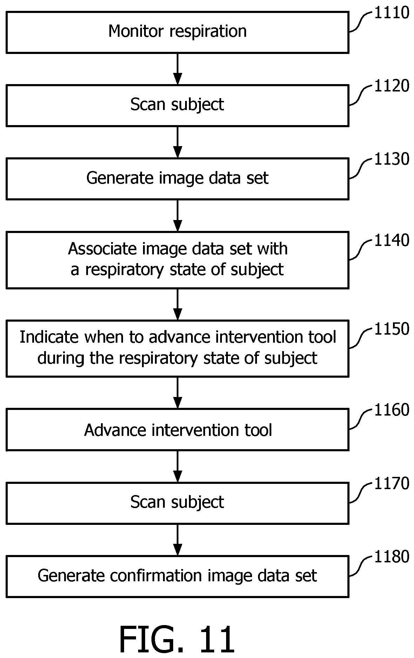

FIGURE 11 is a flowchart of an exemplary method of advancing an intervention tool while the patient can breathe normally and continuously, without holding his breath. Steps 1110-1150 are similar to steps 1010-1050 mentioned above. At step 1160 a user can advance the intervention tool based on the indication from the intervention tool advancement indicator from step 1150. Steps 1150 and 1160 may be repeated any number of times to advance the intervention tool a certain distance before re-scanning the patient, depending on any number of factors, including, for example, the nature of the intervention procedure, the proximity of the intervention tool to other sensitive areas of the patient, the amount of incremental advancement of the intervention tool during each advancement step 1160, the amount of radiation the patient is exposed to during each scan, etc. Next, the patient can be re-scanned using the scanner at step 1170 and a confirmation image data set is generated from the scan at step 1180. The image data set from step 1180 may also be used as a new data set replacing the data set from step 1130 and continuing the method with step 1140. In this manner, a user can advance the intervention tool knowing that the patient's target area is in the same position as when the scan was taken, the patient does not need to hold his breath, and the intervention procedure can occur with a minimum amount of radiation exposure to the patient.

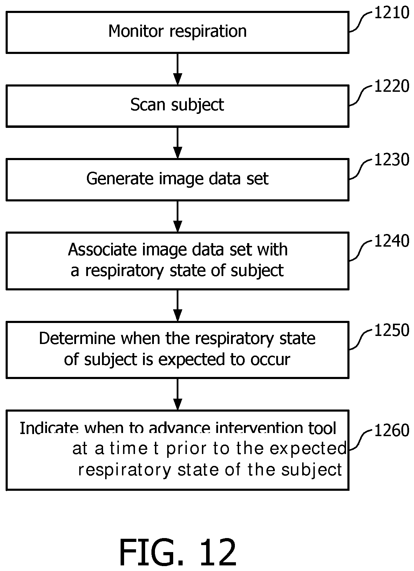

FIGURE 12 is a flowchart of another exemplary method of indicating when to advance an intervention tool while the patient can breathe normally and continuously, without holding his breath. Steps 1210-1240 are similar to steps 1010-1040 mentioned above. At step 1250 a processor can determine when the target respiratory state of the patient is expected to occur using various algorithms, including those mentioned above. At step 1260, while continuing to monitor the patient's respiration, an intervention tool advancement indicator can indicate when to advance an intervention tool at a time t prior to the expected subsequent respiratory state of the patient that matches the respiratory state of the image data set. In this manner, an advancement indicator can provide a user with an indication of when to advance the intervention tool at a time t before when the patient's target area is in the same position as when the scan was taken.

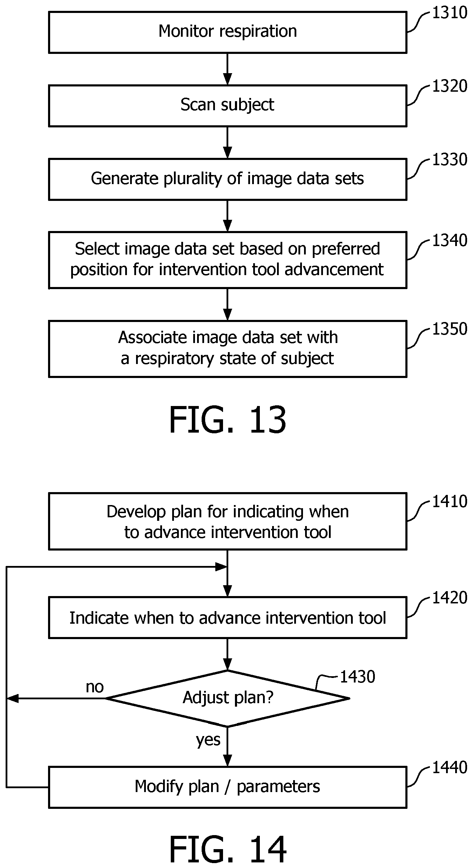

FIGURE 13 is a flowchart of an exemplary method of selecting an image data set for use during an intervention procedure. Steps 1310-1320 are similar to steps 1010-1020 mentioned above. At step 1330 a plurality of image data sets are generated from the scan at step 1320. The plurality of image data sets may correspond to a plurality of respiration states of the patient. At step 1340, one of the plurality of image data sets may be selected as the preferred position of the patient during the interventional procedure. At step 1350, the selected image data set is associated with the respiratory state of the patient at the time of the scan that produced the selected image data set.

FIGURE 14 is a flowchart of an exemplary method of developing a plan for indicating when to advance an intervention tool. At step 1410, during a planning phase, parameters associated with when and for how long an intervention tool advancement indicator should indicate when to advance the intervention tool are determined. Next, at step 1420, an intervention tool advancement indicator indicates when to advance the intervention tool according to the plan. At step 1430, a determination is made whether to adjust the plan based on various factors, such as, for example, detected organ movement, the location of the intervention tool, or a user selection. A confirmation scan may be used to assist in determining whether to adjust the plan and how to adjust the plan. If modifications are not required, the method continues according to the previous plan. If modifications are required, the plan and its associated parameters may be modified before proceeding. In this manner, the advancement indicator can indicate when to advance the intervention tool according to an intervention procedure plan.

While the present invention has been illustrated by the description of embodiments thereof, and while the embodiments have been described in some detail, it is not the intention of the applicant to restrict or in any way limit the scope of the appended claims to such detail. Additional advantages and modifications will readily appear to those skilled in the art. The invention may take form in various compositions, components and arrangements, combinations and sub-combinations of the elements of the disclosed embodiments. Therefore, the invention in its broader aspects is not limited to the specific details, representative apparatus and methods, and illustrative examples shown and described. Accordingly, departures may be made from such details without departing from the scope of the applicant's general inventive concept.

Having thus described several embodiments, the invention is now claimed to be:

## Claims

1. A respiratory monitoring method for indicating intervention tool advancement timing during an intervention procedure while respiration of a subject is continuous, the method comprising:
monitoring (1010) respiration of the subject using a respiration monitor (160), wherein the respiration monitor (160) produces a respiratory signal indicative of a plurality of respiratory states of the subject;
generating (1030) a first imaging data set from a scan of the subject with an imaging device (100), wherein the first imaging data set is associated with a first respiratory state of the subject; and
indicating (1050) when to advance an intervention tool based on the respiratory signal using an advancement indicator (150), **characterised in that** the step of indicating (1050) when to advance the intervention tool occurs at a predetermined time prior to the first respiratory state of the subject, the method comprising determining when the first respiratory state of the subject is expected to occur, wherein the step of determining when the first respiratory state of the subject is expected to occur comprises determining a slope of the respiratory signal.

2. The method of claim 1, wherein the step of indicating (1050) when to advance the intervention tool occurs only when the subject is exhaling.

3. The method of claim 1, wherein the step of indicating (1050) when to advance the intervention tool occurs only when the subject is inhaling.

4. The method of claim 1, further comprising:
generating a second imaging data set, wherein the second imaging data set is associated with a second respiratory state of the subject; and
indicating when to advance the intervention tool based on the respiratory signal using
the advancement indicator (150), such that the intervention tool can be advanced during the second respiratory state of the subject.

5. The method of claim 1, wherein the intervention tool can be advanced automatically.

6. The method of claim 1, further comprising:
generating (1180) a confirmation imaging data set to confirm the location of the intervention tool.

7. The method of claim 1, further comprising:
generating (1330) a plurality of imaging data sets, wherein the plurality of imaging data sets are associated with a plurality of respiratory states of the subject; and
selecting (1340) the first imaging data set from the plurality of imaging data sets, wherein the first imaging data set is associated with a preferred position of the subject during intervention tool advancement.

8. The method of claim 1, further comprising:
developing (1410) a plan for indicating when to advance the intervention tool based on the respiratory signal using the advancement indicator (150), wherein the plan includes establishing parameters associated with the advancement indicator.

9. The method of claim 8, further comprising:
modifying (1440) the plan for indicating when to advance the intervention tool based on the progress of the intervention procedure.

10. The method of claim 1, further comprising:
determining a length of time for indicating when to advance the intervention tool.

11. The method of claim 10, wherein determining the length of time for indicating when to advance the intervention tool is based on at least one of detecting organ movement, a location of the intervention tool, and a user selection.

12. A respiratory monitoring system for indicating intervention tool advancement timing during an intervention procedure while a subject is not holding his or her breath, comprising:
a respiration monitor (160) configured for monitoring respiration of the subject, wherein the respiration monitor (160) is configured for producing a respiratory signal indicative of a plurality of respiratory states of the subject;
an imaging device (100) configured for scanning the subject and generating a first imaging data set, wherein the first imaging data set is associated with a first respiratory state of the subject; and
an advancement indicator (150) configured for indicating when to advance an intervention tool based on the respiratory signal, such that advancement of the intervention tool occurs during the first respiratory state of the subject, **characterised in that** the advancement indicator (150) is configured for indicating when to advance the intervention tool at a predetermined time prior to the first respiratory state of the subject, the respiratory monitoring system comprising means configured for determining when the first respiratory state of the subject is expected to occur, wherein the means is configured for determining a slope of the respiratory signal for determining when the first respiratory state of the subject is expected to occur.

13. The system of claim 12, wherein the respiration monitors (160) comprises at least one of a bellows belt, an external marker, and a motion based pressure sensor associated with the subject.

14. The system of claim 12, wherein the respiration monitor (160) is configured for sensing at least one of a position, a motion, a pressure, and an air volume associated with the subject.

15. The system of claim 12, wherein the advancement indicator (150) comprises at least one of an audio device and a video device for indicating when to advance the intervention tool.

## Patentansprüche

1. Atemüberwachungsverfahren zum Angeben der Zeiten zum Vorschieben eines Interventionsinstruments während einer Interventionsprozedur während der kontinuierlichen Atmung eines Patienten, wobei das Verfahren Folgendes umfasst:
Überwachen (1010) der Atmung des Patienten unter Verwendung eines Atmungsmonitors (160), wobei der Atmungsmonitor (160) ein Atmungssignal erzeugt, das eine Vielzahl von Atmungsphasen des Patienten angibt;
Erzeugen (1030) eines ersten Bildgebungsdatensatzes aus einem Scan des Patienten mit einer Bildgebungsvorrichtung (100), wobei der erste Bildgebungsdatensatz zu einer ersten Atmungsphase des Patienten gehörig ist; und
Angeben (1050), basierend auf dem Atmungssignal unter Verwendung eines Vorschiebungsindikators (150), wann das Interventionsinstrument vorzuschieben ist, **dadurch gekennzeichnet, dass** der Schritt des Angebens (1050), wann das Interventionsinstrument vorzuschieben ist, zu einem vorgegebenen Zeitpunkt vor der ersten Atmungsphase des Patienten erfolgt,
wobei das Verfahren Folgendes umfasst:
Ermitteln, wann die erste Atmungsphase des Patienten voraussichtlich auftreten wird, wobei der Schritt des Ermittelns, wann die erste Atmungsphase des Patienten voraussichtlich auftreten wird, das Ermitteln einer Steigung des Atmungssignals umfasst.

2. Verfahren nach Anspruch 1, wobei der Schritt des Angebens (1050), wann das Interventionsinstrument vorzuschieben ist, nur erfolgt, wenn der Patient ausatmet.

3. Verfahren nach Anspruch 1, wobei der Schritt des Angebens (1050), wann das Interventionsinstrument vorzuschieben ist, nur erfolgt, wenn der Patient einatmet.

4. Verfahren nach Anspruch 1, weiterhin umfassend:
Erzeugen eines zweiten Bildgebungsdatensatzes, wobei der zweite Bildgebungsdatensatz zu einer zweiten Atmungsphase des Patienten gehörig ist; und
Angeben, basierend auf dem Atmungssignal unter Verwendung des Vorschiebungsindikators (150), wann das Interventionsinstrument vorzuschieben ist, sodass das Interventionsinstrument während der zweiten Atmungsphase des Patienten vorgeschoben werden kann.

5. Verfahren nach Anspruch 1, wobei das Interventionsinstrument automatisch vorgeschoben werden kann.

6. Verfahren nach Anspruch 1, weiterhin umfassend:
Erzeugen (1180) eines Bestätigungsbildgebungsdatensatzes, um den Ort des Interventionsinstruments zu bestätigen.

7. Verfahren nach Anspruch 1, weiterhin umfassend:
Erzeugen (1330) einer Vielzahl von Bildgebungsdatensätzen, wobei die Vielzahl von Bildgebungsdatensätzen zu einer Vielzahl von Atmungsphasen des Patienten gehörig ist; und
Auswählen (1340) des ersten Bildgebungsdatensatzes aus der Vielzahl von Bildgebungsdatensätzen, wobei der erste Bildgebungsdatensatz zu einer bevorzugten Position des Patienten während des Vorschiebens des Interventionsinstruments gehörig ist.

8. Verfahren nach Anspruch 1, weiterhin umfassend:
Entwickeln (1410) eines Plans zum Angeben, basierend auf dem Atmungssignal unter Verwendung des Vorschiebungsindikators (150), wann das Interventionsinstrument vorzuschieben ist, wobei der Plan das Aufstellen von zu dem Vorschiebungsindikator gehörigen Parametern umfasst.

9. Verfahren nach Anspruch 8, weiterhin umfassend:
Modifizieren (1440) des Plans zum Angeben, wann das Interventionsinstrument vorzuschieben ist, basierend auf dem Fortschritt der Interventionsprozedur.

10. Verfahren nach Anspruch 1, weiterhin umfassend:
Ermitteln einer Zeitdauer für das Angeben, wann das Interventionsinstrument vorzuschieben ist.

11. Verfahren nach Anspruch 10, wobei das Ermitteln der Zeitdauer für das Angeben, wann das Interventionsinstrument vorzuschieben ist, auf mindestens entweder dem Detektieren einer Organbewegung, einem Ort des Interventionsinstruments oder einer Benutzerauswahl basiert.

12. Atemüberwachungssystem zum Angeben der Zeiten zum Vorschieben eines Interventionsinstruments während einer Interventionsprozedur während ein Patient seinen Atem nicht anhält, umfassend:
einen Atmungsmonitor (160), der dafür konfiguriert ist, die Atmung des Patienten zu überwachen, wobei der Atmungsmonitor (160) dafür konfiguriert ist, ein Atmungssignal zu erzeugen, das eine Vielzahl von Atmungsphasen des Patienten angibt;
eine Bildgebungsvorrichtung (100), die dafür konfiguriert ist, den Patienten zu scannen und einen ersten Bildgebungsdatensatz zu erzeugen, wobei der erste Bildgebungsdatensatz zu einer ersten Atmungsphase des Patienten gehörig ist; und
einen Vorschiebungsindikator (150), der dafür konfiguriert ist, basierend auf dem Atmungssignal anzugeben, wann das Interventionsinstrument vorzuschieben ist, sodass das Vorschieben des Interventionsinstruments während der ersten Atmungsphase des Patienten erfolgt, **dadurch gekennzeichnet, dass** der Vorschiebungsindikator (150) konfiguriert ist, um zu einem vorgegebenen Zeitpunkt vor der ersten Atmungsphase des Patienten anzugeben, wann das Interventionsinstrument vorzuschieben ist,
wobei das Atmungsüberwachungssystem Mittel umfasst, die konfiguriert sind, um zu ermitteln, wann die erste Atmungsphase des Patienten voraussichtlich auftreten wird, wobei die Mittel konfiguriert sind, um eine Steigung des Atmungssignals zu ermitteln, um zu ermitteln, wann die erste Atmungsphase des Patienten voraussichtlich auftreten wird

13. System nach Anspruch 12, wobei der Atmungsmonitor (160) mindestens entweder einen Atemgurt, eine externe Markierung oder einen bewegungsbasierten Drucksensor umfasst, die dem Patienten zugehörig sind.

14. System nach Anspruch 12, wobei der Atmungsmonitor (160) dafür konfiguriert ist, mindestens entweder eine Position, eine Bewegung, einen Druck oder ein Luftvolumen zu erfassen, die dem Patienten zugehörig sind.

15. System nach Anspruch 12, wobei der Vorschiebungsindikator (150) mindestens entweder eine Audiovorrichtung oder eine Videovorrichtung zum Angeben des Zeitpunkts zum Vorschieben des Interventionsinstruments umfasst.

## Revendications

1. Procédé de surveillance respiratoire pour indiquer le synchronisme d'avancement d'un outil d'intervention au cours d'une procédure d'intervention tandis que la respiration d'un sujet est continue, le procédé comprenant les étapes consistant à :
surveiller (1010) la respiration du sujet en utilisant un moniteur de respiration (160), dans lequel le moniteur de respiration (160) produit un signal respiratoire indicatif d'une pluralité d'états respiratoires du sujet ;
générer (1030) un premier ensemble de données d'imagerie à partir d'un balayage du sujet par un dispositif d'imagerie (100), dans lequel le premier ensemble de données d'imagerie est associé à un premier état respiratoire du sujet ; et
indiquer (1050) à quel moment il faut avancer un outil d'intervention sur la base du signal respiratoire en utilisant un indicateur d'avancement (150), **caractérisé en ce que** l'étape d'indication (1050) du moment où il faut avancer l'outil d'intervention se produit à un moment prédéterminé avant le premier état respiratoire du sujet,
le procédé comprenant :
la détermination du moment où le premier état respiratoire du sujet est censé se produire, dans lequel l'étape de détermination du moment où le premier étant respiratoire du sujet est censé se produire comprend la détermination d'une pente du signal respiration.

2. Procédé selon la revendication 1, dans lequel l'étape d'indication (1050) du moment où il faut avancer l'outil d'intervention se produit uniquement lorsque le sujet exhale.

3. Procédé selon la revendication 1, dans lequel l'étape d'indication (1050) du moment où il faut avancer l'outil d'intervention se produit uniquement lorsque le sujet inhale.

4. Procédé selon la revendication 1, comprenant en outre :
la génération d'un second ensemble de données d'imagerie, dans lequel le second ensemble de données d'imagerie est associé à un second état respiratoire du sujet ; et
l'indication du moment où il faut avancer l'outil d'intervention sur la base du signal respiratoire en utilisant l'indicateur d'avancement (150) de sorte que l'outil d'intervention puisse être avancé au cours du second état respiratoire du sujet.

5. Procédé selon la revendication 1, dans lequel l'outil d'intervention peut être avancé automatiquement.

6. Procédé selon la revendication 1, comprenant en outre :
la génération (1180) d'un ensemble de données d'imagerie de confirmation pour confirmer l'emplacement de l'outil d'intervention.

7. Procédé selon la revendication 1, comprenant en outre :
la génération (1330) d'une pluralité d'ensembles de données d'imagerie, dans lequel la pluralité d'ensembles de données d'imagerie est associée à une pluralité d'états respiratoires du sujet ; et
la sélection (1340) du premier ensemble de données d'imagerie parmi la pluralité d'ensembles de données d'imagerie, dans lequel le premier ensemble de données d'imagerie est associé à une position préférée du sujet au cours de l'avancement de l'outil d'intervention.

8. Procédé selon la revendication 1, comprenant en outre :
le développement (1410) d'un plan pour indiquer à quel moment il faut avancer l'outil d'intervention sur la base du signal respiratoire en utilisant l'indicateur d'avancement (150), dans lequel le plan comprend l'établissement de paramètres associés à l'indicateur d'avancement.

9. Procédé selon la revendication 8, comprenant en outre :
la modification (1440) du plan pour indiquer à quel moment il faut avancer l'outil d'intervention sur la base de la progression de la procédure d'intervention.

10. Procédé selon la revendication 1, comprenant en outre :
la détermination d'une période de temps pour indiquer à quel moment il faut avancer l'outil d'intervention.

11. Procédé selon la revendication 10, dans lequel la détermination de la période de temps pour indiquer à quel moment il faut avancer l'outil d'intervention est basée sur au moins l'un(e) parmi la détection d'un mouvement d'organe, d'un emplacement de l'outil d'intervention et d'une sélection de l'utilisateur.

12. Système de surveillance respiratoire pour indiquer le synchronisme d'avancement de l'outil d'intervention au cours d'une procédure d'intervention tandis qu'un sujet ne retient pas sa respiration, comprenant :
un moniteur de respiration (160) configuré pour produire un signal respiratoire indicatif d'une pluralité d'états respiratoires du sujet ; dans lequel le moniteur de respiration (160) est configuré pour produire un signal respiratoire indicatif d'une pluralité d'états respiratoires du sujet ;
un dispositif d'imagerie (100) configuré pour balayer le sujet et générer un premier ensemble de données d'imagerie, dans lequel le premier ensemble de données d'imagerie est associé à un premier état respiratoire du sujet ; et
un indicateur d'avancement (150) configuré pour indiquer à quel moment il faut avancer un outil d'intervention sur la base du signal respiratoire de sorte que l'avancement de l'outil d'intervention se produise au cours du premier état respiratoire du sujet, **caractérisé en ce que** l'indicateur d'avancement (150) est configuré pour indiquer à quel moment il faut avancer l'outil d'intervention à un moment prédéterminé avant le premier état respiratoire du sujet,
le système de surveillance respiratoire comprenant des moyens configurés pour déterminer à quel moment le premier état respiratoire du sujet est censé se produire, dans lequel les moyens sont configurés pour déterminer une pente du signal respiratoire afin de déterminer à quel moment le premier état respiratoire du sujet est censé se produire.

13. Système selon la revendication 12, dans lequel le moniteur de respiration (160) comprend au moins l'un(e) d'une ceinture de soufflet, d'un marqueur externe et d'un capteur de pression à base de mouvement associés au sujet.

14. Système selon la revendication 12, dans lequel le moniteur de respiration (160) est configuré pour détecter au moins l'un(e) d'une position, d'un mouvement, d'une pression et d'un volume d'air associés au sujet.

15. Système selon la revendication 12, dans lequel l'indicateur d'avancement (150) comprend au moins l'un d'un dispositif audio et d'un dispositif vidéo pour indiquer à quel moment il faut avancer l'outil d'intervention.
